# EUROPEAN PATENT SPECIFICATION

(11) **EP 1 829 538 B1**
(45) Date of publication and mention of the grant of the patent: **20.01.2016**
(21) Application number: 05819588.4
(22) Date of filing: 22.12.2005
(51) Int. Cl.: A61K 31/122, A61K 9/56, A61P 3/02, A61K 9/28

(54) **SOLID PREPARATION COMPRISING REDUCED COENZYME Q10 AND PROCESS FOR PRODUCTION OF THE SAME**
FESTE ZUBEREITUNG MIT REDUZIERTEM COENZYM Q10 UND VERFAHREN ZU IHRER HERSTELLUNG
PREPARATION SOLIDE COMPRENANT LA COENZYME Q10 REDUITE ET PROCEDE POUR LA PRODUCTION DE CELLE-CI

(30) Priority: 24.12.2004 JP 2004373553
(43) Date of publication of application: 05.09.2007
(73) Proprietor: KANEKA CORPORATION, Osaka (JP)
(72) Inventor: ONO, Tadao KANEKA CORPORATION Takasago Plant, Hyogo 6768688 (JP); UEDA, Takahiro KANEKA CORPORATION Takasago Plant, Hyogo 6768688 (JP); KITAMURA, Shiro KANEKA CORPORATION Takasago Plant, Hyogo 6768688 (JP); UEDA, Yasuyoshi KANEKA CORPORATION Takasago Plant, Hyogo 6768688 (JP)
(74) Representative: Hart-Davis, Jason
(86) International application number: PCT/JP2005/023623
(87) International publication number: WO 2006/075502

(56) References cited:
- EP-A1- 1 440 962
- WO-A1-01/52822
- WO-A1-98/07417
- WO-A1-03/032967
- WO-A1-03/077895
- JP-A- 11 127 785
- JP-A- 56 020 513
- JP-A- 2000 302 672
- JP-A- 2003 026 625
- JP-A- 2003 119 127
- JP-A- 2003 246 733

## Description

### TECHNICAL FIELD

This invention relates to a solid preparation containing reduced coenzyme Q₁₀, a method for producing the same, a method for stabilizing a solid preparation containing reduced coenzyme Q₁₀, and a method of handling the same.

### BACKGROUND ART

Reduced coenzyme Q₁₀ shows higher oral absorbability as compared with oxidized coenzyme Q₁₀ and is a favorable compound suited for use in foods, functional nutritive foods, specific health foods, nutritional supplements, nutrients, animal drugs, drinks, feeds, cosmetics, medicines, remedies, preventive drugs, etc.

Several methods have been disclosed in the prior art literature for preparing reduced coenzyme Q₁₀ (WO03/006408, WO03/006409, WO03/006410, WO03/006411, WO03/006412, WO03/008363 and WO03/032967). However, reduced coenzyme Q₁₀ is readily oxidized to oxidized coenzyme Q₁₀ by molecular oxygen, and it is still an important problem to stabilize reduced coenzyme Q₁₀ in processing the same in foods, functional nutritive foods, specific health foods, nutritional supplements, nutrients, animal drugs, drinks, feeds, cosmetics, medicines, remedies, preventive drugs, etc., or raw materials or compositions for the production thereof, and/or to stabilize the same in handling such products, raw materials or compositions after incorporation of the same.

On the occasion of such handling, it is very difficult to completely eliminate or shut out oxygen and, in particular in the step of warming for processing or during long-period preservation of such products, the remaining or contaminant oxygen exerts a great adverse influence and is directly concerned with such quality problems as the formation of oxidized coenzyme Q₁₀ as a byproduct.

Thus, it is a very important problem to stabilize (protect against oxidation) reduced coenzyme Q₁₀. To this time, however, few studies have been made on the method and composition for stabilizing coenzyme Q₁₀. There are only two examples; one describes a composition comprising a coexisting reducing agent and a method for producing the same (WO01/052822) and, in the other, reduced coenzyme Q₁₀ is stabilized in an oil or fat (WO03/062182).

In WO01/052822, there are disclosed 1) a composition comprising an amount, effective in preventing reduced coenzyme Q₁₀ from being oxidized to oxidized coenzyme Q₁₀, of a reducing agent and an amount, effective in dissolving the reduced coenzyme Q₁₀ and reducing agent, of a surfactant or a vegetable oil or a mixture of these, if necessary together with a solvent, 2) a composition for oral administration in the form of gelatin capsule or tablets as prepared from the above composition and, further, 3) a method for preparing the above composition containing reduced coenzyme Q₁₀ in situ by using oxidized coenzyme Q₁₀ and a reducing agent.

In WO01/052822, however, there is no detailed description of the quality of the reduced coenzyme Q₁₀ contained in the above-mentioned compositions or the stabilizing effect, for example. The above-mentioned compositions and the method for preparing the same are very complicated and troublesome so that the compositions may play a plurality of roles (namely the role as a reaction field for the reduction of oxidized coenzyme Q₁₀ to reduced coenzyme Q₁₀ and the role in maintaining reduced coenzyme Q₁₀ in a stable condition). Further, it is known that when an ascorbic acid (reducing agent) is enclosed in gelatin capsules, the disintegrability of the gelatin capsules generally deteriorates, whereby the absorbability in the living body is adversely affected.

Furthermore, it is noteworthy that the safety of the above compositions and/or the method for preparing the same cannot be always be assured since the reaction mixture is used as such. More specifically, the use of an ascorbic acid as a reducing agent in reducing oxidized coenzyme Q₁₀ to reduced coenzyme Q₁₀ results in the oxidation of the ascorbic acid, leading to the formation of a considerable amount of the corresponding dehydroascorbic acid, which contaminates the above-mentioned compositions. Unlike ascorbic acids, dehydroascorbic acids and the decomposition product oxalic acid are highly harmful. For example, they reportedly cause increases in lipid peroxide level and decreases in antioxidant level in the liver and kidney and increases in oxalic acid level in the kidney, and there is a fear of their producing some adverse effects, for example the effects of decreasing the resistance to oxidative stress and readily causing ureterolithiasis.

In WO03/062182, on the other hand, a method for stabilizing reduced coenzyme Q₁₀ which is characterized in that reduced coenzyme Q₁₀ is incorporated in a composition whose main component is a fat or oil (except for olive oil) and/or a polyol and which will not substantially interfere with the stabilization of reduced coenzyme Q₁₀ is disclosed as a method for protecting reduced coenzyme Q₁₀ against oxidation. However, the above stabilization method may not enough to increase the stability of reduced coenzyme Q₁₀ to a satisfactory extent in some instances. Like in the case of WO01/052822, the above method uses a fat or oil and/or a surfactant and, therefore, the range of application thereof is limited.

WO 03/077895 A1 discloses a composition containing a coenzyme Q as the main component that improves the impaired glucose tolerance of diabetes patients.

JP 2003 026625 A discloses a solution of reduced coenzyme Q prepared by coating reduced coenzyme Q with liposome made of refined soybean lecithin.

### SUMMARY OF THE INVENTION

In view of the above-mentioned state of the art, it is an object of the present invention to provide a solid preparation containing highly stabilized reduced coenzyme Q₁₀ and a method for producing the same as well as a method for stabilizing such a solid preparation containing reduced

coenzyme Q₁₀ and a method for handling the same to be used or practiced in the fields of foods, functional nutritive foods, specific health foods, nutritional supplements, nutrients, animal drugs, drinks, feeds, cosmetics, medicines, remedies, preventive drugs, etc.

The present inventors made intensive investigations in an attempt to accomplish the above object and, as a result, found that a solid preparation containing reduced coenzyme Q₁₀ coated with at least one coating medium selected from among oil-soluble coating media and water-soluble coating media can be protected in a surprisingly favorable manner against the oxidation, by molecular oxygen, of reduced coenzyme Q₁₀ in the solid preparation. Based on such finding, they have now completed the present invention.

The subject-matter of the present invention is defined in the claims.

Thus, the disclosure provides the following:
(1) A solid preparation containing reduced coenzyme Q₁₀ which comprises a solid composition containing reduced coenzyme Q₁₀ coated with at least one coating medium selected from among oil-soluble coating media and water-soluble coating media.
(2) The solid preparation according to (1),
   wherein the solid composition containing reduced coenzyme Q₁₀ is coated with a water-soluble coating medium and then further with an oil-soluble coating medium.
(3) The solid preparation according to (1),
   wherein the coating medium is that accepted for use in foods.
(4) The solid preparation according to (1),
   wherein the coating medium used is a water-soluble coating medium.
(5) The solid preparation according to (1),
   wherein the oil-soluble coating medium used is at least one species selected from among shellac and zein.
(6) The solid preparation according to (1),
   wherein the water-soluble coating medium used is at least one species selected from the group consisting of gelatin and yeast cell wall fractions.
(7) The solid preparation according to (1),
   wherein the total coating weight of the coating medium or media is not less than 5% by weight but not more than 99.9% by weight relative to the weight of the solid preparation.
(8) The solid preparation according to (1),
   which shows a percent retention of reduced coenzyme Q₁₀ of not lower than 50% by weight after 30 days of preservation in the air at 40°C in a condition protected from light.
(9) A method for producing a solid preparation containing reduced coenzyme Q₁₀,
   wherein a solid composition containing reduced coenzyme Q₁₀ is coated with at least one coating medium selected from among oil-soluble coating media and water-soluble coating media.
(10) The production method according to (9),
   wherein the solid composition containing reduced coenzyme Q₁₀ is coated with a water-soluble coating medium and then further with an oil-soluble coating medium.
(11) The production method according to (9),
   wherein the coating medium is that accepted for use in foods.
(12) The production method according to (9),
   wherein the coating medium used is a water-soluble coating medium.
(13) The production method according to (9),
   wherein the oil-soluble coating medium used is at least one species selected from among shellac and zein.
(14) The production method according to (9),
   wherein the water-soluble coating medium used is at least one species selected from the group consisting of gelatin and yeast cell wall fractions.
(15) The production method according to (9),
   wherein the coating is carried out at a temperature of not lower than 0°C but not higher than 120°C.
(16) A method for stabilizing a solid preparation containing reduced coenzyme Q₁₀,
   wherein a solid composition containing reduced coenzyme Q₁₀ is coated with at least one coating medium selected from among oil-soluble coating media and water-soluble coating media to thereby stabilize the resulting solid preparation containing reduced coenzyme Q₁₀.
(17) The stabilization method according to (16), wherein the solid composition containing reduced coenzyme Q₁₀ is coated with a water-soluble coating medium and then further with an oil-soluble coating medium.
(18) The stabilization method according to (16), wherein the coating medium is that accepted for use in foods.
(19) The stabilization method according to (16), wherein the coating medium used is a water-soluble coating medium.
(20) The stabilization method according to (16), wherein the oil-soluble coating medium used is at least one species selected from among shellac and zein.
(21) The stabilization method according to (16), wherein the water-soluble coating medium used is at least one species selected from the group consisting of gelatin and yeast cell wall fractions.
(22) The stabilization method according to (16), wherein the percent retention of reduced coenzyme Q₁₀ is not lower than 50% by weight after 30 days of preservation in the air at 40°C in a condition protected from light.
(23) A method for handling a solid preparation containing reduced coenzyme Q₁₀,
   wherein the solid preparation containing reduced coenzyme Q₁₀ according to (1) is placed in an environment adjusted to a relative humidity of not higher than 75%.
(24) The handling method according to (23),
   wherein the percent retention of reduced coenzyme Q₁₀ is not lower than 80% by weight after 30 days of preservation in the air at 40ºC in a condition protected from light.

### DETAILED DESCRIPTION OF THE INVENTION

In the following, the present disclosure and invention are described in detail, the invention being defined in the claims.

The term "coenzyme Q₁₀" used herein means both of reduced one and oxidized one, and, in the case where there are both of them, the above term means the mixture as a whole.

First, the solid preparation and the method for producing the same are described.

The solid preparation of the disclosure which contains reduced coenzyme Q₁₀ comprises a solid composition containing reduced coenzyme Q₁₀ as coated with at least one coating medium selected from among oil-soluble coating media and water-soluble coating media.

The method of the disclosure for producing solid preparations containing reduced coenzyme Q₁₀ is characterized in that a solid composition containing reduced coenzyme Q₁₀ is coated with at least one coating medium selected from among oil-soluble coating media and water-soluble coating media.

Reduced coenzyme Q₁₀, which is contained in the solid preparation of the invention is represented by the formula (1): 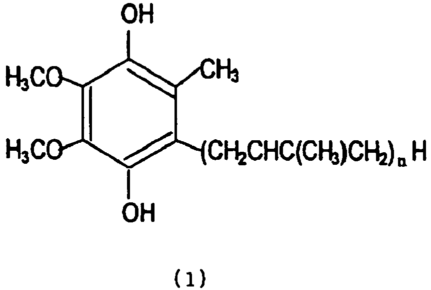 (in which n = 10).

The solid preparation of the invention which contains reduced coenzyme Q₁₀ may contain reduced coenzyme Q₁₀ alone or may further contain oxidized coenzyme Q₁₀.

When the solid preparation contains both reduced coenzyme Q₁₀ and oxidized coenzyme Q₁₀, the proportion of reduced coenzyme Q₁₀ relative to the whole amount of coenzyme Q₁₀ (namely the sum of reduced coenzyme Q₁₀ and oxidized coenzyme Q₁₀) is not particularly restricted but, from the viewpoint of performing the function of reduced coenzyme Q₁₀ markedly, it is, for example not lower than about 20% by weight, generally not lower than about 40% by weight, preferably not lower than about 60% by weight, more preferably not lower than about 80% by weight, still more preferably not lower than about 90% by weight, most preferably not lower than about 96% by weight. The upper limit is, but is not limited to, 100% by weight and generally is not higher than about 99.9% by weight.

The weights of reduced coenzyme Q₁₀ and oxidized coenzyme Q₁₀ can be measured, for example, by the method described in the below-mentioned Examples.

As is described in Japanese Kokai Publication Hei-10-109933, reduced coenzyme Q₁₀ can be produced, for example, by preparing a mixture of oxidized coenzyme Q₁₀ and reduced coenzyme Q₁₀ by such a known method as synthesis, fermentation or extraction from a natural product and subjecting the mixture to chromatography for the concentration of the reduced coenzyme Q₁₀ fraction in the eluate. On that occasion, the oxidized coenzyme Q₁₀ contained together with the reduced coenzyme Q₁₀ may be reduced with an ordinary reducing agent such as sodium borohydride or sodium dithionite, followed by concentration by chromatography. Reduced coenzyme Q₁₀ can also be obtained by reacting an existing high purity grade of coenzyme Q₁₀ with such a reducing agent as mentioned above. Preferably, reduced coenzyme Q₁₀ is obtained by reducing oxidized coenzyme Q₁₀ such as an existing high purity grade of coenzyme Q₁₀, or a mixture of oxidized coenzyme Q₁₀ and reduced coenzyme Q₁₀ with an ordinary reducing agent such as sodium dithionite, sodium borohydride or ascorbic acids. More preferably, reduced coenzyme Q₁₀ is obtained by reducing oxidized coenzyme Q₁₀ such as an existing high purity grade of coenzyme Q₁₀, or a mixture of oxidized coenzyme Q₁₀ and reduced coenzyme Q₁₀ with ascorbic acids.

The solid composition prior to coating with at least one coating medium selected from among oil-soluble coating media and water-soluble coating media is not particularly restricted provided that it contains reduced coenzyme Q₁₀.

As disclosed herein, it is also possible to prepare the solid composition using reduced coenzyme Q₁₀ alone. In that case, reduced coenzyme Q₁₀ as such in the form of a powder or granules may be used as the solid composition for coating with a coating medium.

It is also possible to make the solid composition into dosage forms suited for oral administration, for example powders, fine granules, granules, pills, tablets, hard capsules and soft capsules, according to the known methods for producing pharmaceutical preparations (e.g. the methods described in the Japanese Pharmacopoeia, 14th edition, General rules for preparations) using one or more additives accepted for use in foods, cosmetics and drugs. Chewable tablets may be mentioned as a preferred tablet form.

The solid composition may also be prepared by admixing the above-mentioned additive(s) with a reduced coenzyme Q₁₀-containing granular powder prepared by any of the generally employed methods of granulation (e.g. wet granulation methods such as spray granulation method, tumbling granulation method, extrusion granulation method and fluidized bed granulation method using a solution or dispersion containing water and/or an organic solvent; dry granulation methods such as fluidized bed granulation and tumbling granulation method using a powder binder) and compression molding the resulting mixture.

The additives mentioned above are not particularly restricted but include those accepted for use in foods, cosmetics and drugs. Those accepted for use in foods are particularly preferred. The additives include excipients, disintegrating agents, lubricants, binders, coloring agents, agglomeration inhibitors, absorption promoters, dissolution aids, stabilizers, oils and fats, surfactants, and the like. It is of course possible to incorporate one or more active components other than reduced coenzyme Q₁₀ in the solid composition. These additives may be used singly or two or more of them may be used in combination.

The excipients are not particularly restricted but include, for example, sucrose (purified sucrose, white soft sugar), lactose, glucose, starch, cornstarch, mannitol, crystalline cellulose, calcium phosphate, calcium sulfate and the like.

The disintegrating agents are not particularly restricted but include, for example, starch, agar, calcium citrate, calcium carbonate, sodium hydrogen carbonate, dextrin, crystalline cellulose, carboxymethylcellulose, tragacanth, alginic acid and the like.

The lubricants are not particularly restricted but include, for example, talc, magnesium stearate, polyethylene glycol, silica, hydrogenated oils and the like.

The binders are not particularly restricted but include, for example, ethylcellulose, methylcellulose, hydroxypropylmethylcellulose, hydroxypropylcellulose, tragacanth, shellac, gelatin, pullulan, gum arabic, polyvinylpyrrolidone, polyvinylalcohol, polyacrylic acid, polymethacrylic acid, sorbitol and the like.

The coloring agents are not particularly restricted but include, for example, titanium oxide, food colors, bengal colors, safflower colors, caramel colors, gardenia colors, tar colors, chlorophyll and the like.

The agglomeration inhibitors are not particularly restricted but include, for example, stearic acid, talc, light anhydrous silicic acid, hydrous silicon dioxide and the like.

The absorption promoters are not particularly restricted but include, for example, higher alcohols, higher fatty acids and the like.

The dissolution aids are not particularly restricted but include, for example, organic acids such as fumaric acid, succinic acid, malic acid and the like.

The stabilizers are not particularly restricted but include, for example, benzoic acid, sodium benzoate, ethyl parahydroxybenzoate, beeswax and the like.

The oils and fats are not particularly restricted but may be, for example, natural oils and fats derived from animals or plants, synthetic oils and fats, or modified oils and fats. More preferred are those accepted for use in foods, cosmetics or drugs. As the vegetable oils and fats, there may be mentioned, for example, coconut oil, palm oil, palm kernel oil, linseed oil, camellia oil, brown rice germ oil, rapeseed oil, rice oil, peanut oil, corn oil, wheat germ oil, soybean oil, perillan oil, cotton seed oil, sunflower seed oil, kapok oil, evening primrose oil, shea butter, sal fat, cacao butter, sesame oil, safflower oil, olive oil and the like. As the animal-derived oils and fats, there may be mentioned, for example, lard, milk fat, fish oils, beef tallow and the like. Moreover, there may also be mentioned modified oils and fats obtainable by the fractionation, hydrogenation, transesterificaiton, etc. of these natural oils and fats (e.g. hydrogenated oils). It is of course possible to use medium chain fatty acid triglycerides (MCTs). Mixtures of these may also be used.

As the medium chain fatty acid triglycerides, there may be mentioned, for example, triglycerides whose fatty acid-derived moieties each contain 6 to 12 carbon atoms, preferably 8 to 12 carbon atoms.

Among the oils and fats enumerated above, vegetable oils and fats, synthetic oils and fats, and modified oils and fats, for example, are preferred from the easy handing and odor viewpoint. As examples, there may be mentioned coconut oil, palm oil, palm kernel oil, rapeseed oil, rice oil, soybean oil, cotton seed oil, safflower oil, olive oil, MCTs and the like.

As the surfactants, there may be mentioned, for example, fatty acid partial glycerides, propylene glycol fatty acid esters, phospholipids, sucrose fatty acid esters, sorbitan fatty acid esters, polyoxyethylenesorbitan fatty acid esters, polyglycerol fatty acid esters and the like.

As the fatty acid partial glycerides, there may be mentioned, for example, monoglycerides and diglycerides derived from fatty acids each containing 6 to 18 carbon atoms, preferably 6 to 12 carbon atoms.

As the propylene glycol fatty acid esters, there may be mentioned, for example, monoesters and diesters derived from fatty acids each containing 6 to 18 carbon atoms, preferably 6 to 12 carbon atoms.

As the phospholipids, there maybe mentioned, for example, egg yolk lecithin, purified soybean lecithin, phosphatidylcholine, phosphatidylethanolamine, phosphatidylserine, sphingomyelin, dicetyl phosphate, stearylamine, phosphatidylglycerol, phosphatidic acid, phosphatidylinositolamine, cardiolipin, ceramide phosphorylethanolamine, ceramide phosphorylglycerol, and mixtures of these, and the like.

As the sucrose fatty acid esters, sorbitan fatty acid esters, polyoxyethylenesorbitan fatty acid esters and polyglycerol fatty acid esters, there may be mentioned, for example, ones derived from fatty acids each containing 6 or more carbon atoms, preferably 8 or more carbon atoms.

The other active components are not particularly restricted provided that they are accepted for use in foods, cosmetics or drugs. Thus, there may be mentioned, amino acids, vitamins, minerals, polyphenols, organic acids, sugars, peptides, proteins and the like. Among these, the ones having an antioxidant activity, for example, glutathione, L-cysteine, N-acetylcysteine, reduced α-lipoic acid, tocotrienol, vitamin E (α-tocopherol) and ester derivatives thereof, vitamin C (ascorbic acid) and ester derivatives and salts thereof, erythorbic acid and ester derivatives and salts thereof, vitamin A and ester derivatives thereof, carotenoids, rutin, zeaxanthin, astaxanthin, lycopene, flavonoids, L-carnitine and pharmacologically acceptable salts thereof (e.g. tartrate, fumarate), acetyl-L-carnitine, propionyl-L-carnitine, magnesium, zinc, selenium, manganese, riboflavin, niacinamide, curcuminoids, proanthocyanidine extracted from the grape seed or pine tree bark, NADH (reduced nicotinamide adenine dinucleotide), NADPH (reduced nicotinamide adenine dinucleotide phosphate), resveratrol, a bilberry extract, a milk thistle extract, highly unsaturated fatty acids obtained by concentration of fish oils or the like, and the like are particularly preferred.

Preferred are glutathione, L-cysteine, tocotrienol, vitamin E (α-tocopherol) and ester derivatives thereof, vitamin C (ascorbic acid) and ester derivatives and salts thereof, erythorbic acid and ester derivatives and salts thereof, vitamin A and ester derivatives thereof, carotenoids, rutin, astaxanthin, lycopene, flavonoids, L-carnitine and the like. Preferred among these from the viewpoint of stabilization of reduced coenzyme Q₁₀ are carotenoids, astaxanthin, vitamin E and ester derivatives thereof, vitamin C and ester derivatives and salts thereof, and like antioxidants. It is of course possible to use two or more of the active components mentioned above in admixture.

In accordance with the disclosure, the above-mentioned solid composition is coated using at least one coating medium selected from among oil-soluble coating media and water-soluble coating media so that reduced coenzyme Q₁₀ occurring in the solid composition may be inhibited from being oxidized into oxidized coenzyme Q₁₀ by molecular oxygen.

The coating is preferably other than an enteric coating, considering that the coating should be dissolved in the stomach or the like and coenzyme Q should be absorbed rapidly. As the enteric coating so referred to herein, there may be mentioned compositions containing hypromellose phthalate, diethyl phthalate, polyethylene glycol or a like pharmacologically acceptable enteric coating component.

The oil-soluble coating media in the present invention are not particularly restricted provided that they are soluble in organic solvents other than water, for example the below-mentioned alcohols, ketones, halogenated hydrocarbons, hydrocarbons and the like. The oil-soluble coating media are the ones having solubility (the weight of a solute (% by weight) relative to the weight of a saturated solution) in organic solvents of usually not less than 0.1% by weight, preferably not less than 0.5% by weight, more preferably not less than 1% by weight.

As the oil-soluble coating media, there may be mentioned, for example, higher fatty acid sugar esters, shellac, cellulose derivatives, fatty acids and ester derivatives thereof, and oils and fats, zein and the like. From the viewpoint of stabilization of reduced coenzyme Q₁₀, shellac, cellulose derivatives and zein are preferred, and shellac and zein are more preferred.

As the higher fatty acid sugar esters, there may be mentioned, for example, sucrose palmitate and the like, which have solubility in organic solvents of within the above-mentioned ranges.

As the cellulose derivatives, there may be mentioned, for example, ethylcellulose, methylcellulose, hydroxypropylcellulose, hydroxypropylmethylcellulose, carboxymethylcellulose, and sodium salts (sodium carboxymethylcellose, etc.) and calcium salts (calcium carboxymethylcellose, etc.) thereof, and the like, which have solubility in organic solvents of within the above-mentioned ranges.

As the fatty acids and ester derivatives thereof, there may be mentioned, for example, caprylic acid, capric acid, lauric acid, myristic acid, palmitic acid, stearic acid, oleic acid, behenic acid, and esters thereof (for example, methylesters, ethylesters thereof), and the like.

As the oils and fats, there may be mentioned, for example, the above-mentioned oils and fats. From the viewpoint of maintaining the quality of the solid preparation, preferred are the oils and fats which are in solid state at ordinary temperature.

The water-soluble coating media in the present disclosure are not particularly restricted provided that they are soluble in water. The water-soluble coating media are the ones having solubility (the weight of a solute (% by weight) relative to the weight of a saturated solution) in water of usually not less than 0.1% by weight, preferably not less than 0.5% by weight, more preferably not less than 1% by weight.

As the water-soluble coating media, there may be mentioned, for example, gelatin, sugars, gum arabic, higher fatty acid sugar esters, tragacanth, pectin, pullulan, alginic acid, dried egg white, milk, curdlan, cellulose derivatives, casein, casein compounds, starch, yeast cell wall fractions and the like. From the viewpoint of stabilization of reduced coenzyme Q₁₀, gelatin, sugars, gum arabic, pullulan, cellulose derivatives and yeast cell wall fractions are preferred, gelatin, sugars, cellulose derivatives and yeast cell wall fractions are more preferred, gelatin, cellulose derivatives and yeast cell wall fractions are still more preferred, and yeast cell wall fractions are particularly preferred.

As the sugars, there may be mentioned monosaccharides and disaccharides such as sucrose (purified sucrose, white soft sugar), fructose, glucose, lactose and trehalose, sugar alcohols such as erythritol, mannitol, sorbitol, xylitol, maltitol, powdered reduced maltose syrup and reduced lactose, polysaccharides such as dextrin and maltodextrin, and the like.

As the higher fatty acid sugar esters, there may be mentioned, for example, sucrose palmitate and the like, which have solubility in water of within the above-mentioned ranges.

As the cellulose derivatives, there may be mentioned, for example, ethylcellulose, methylcellulose, hydroxypropylcellulose, hydroxypropylmethylcellulose, carboxymethylcellulose, and sodium salts (sodium carboxymethylcellose, etc.) and calcium salts (calcium carboxymethylcellose, etc.) thereof, and the like, which have solubility in water of within the above-mentioned ranges.

The solubility of the higher fatty acid sugar esters and the cellulose derivatives can be adjusted according to the species of esters, degree of polymerization and the like.

Among the coating media mentioned above, oil-soluble coating media and water-soluble coating media accepted for use in foods are preferred. The term "coating media accepted for use in foods" as used herein means arbitrary nontoxic coating media currently in use in the food industry. Such coating media are not particularly restricted but include gelatin, sugars, gum arabic, pullulan, alginic acid, cellulose derivatives, yeast cell wall fractions, shellac, zein and the like.

Among the coating media mentioned above, water-soluble coating media are preferred from the in vivo disintegration and absorption viewpoint.

Of course, these coating media may be used singly or in the form of a mixture of two or more of them. Two or more coatings can also be made separately.

For increasing the moisture resistance and water resistance of the solid composition, there may be mentioned, for example, the process comprising coating the solid composition first with an oil-soluble coating medium and then further with a water-soluble coating medium. More specifically, the solid composition is preferably coated with shellac or ethylcellulose, for example, and then further coated with gelatin or a yeast cell wall fraction.

For increasing the moisture resistance and water resistance of the solid composition coated with a water-soluble coating medium, there may be mentioned, for example, the process comprising coating the solid composition first with a water-soluble coating medium and then further with an oil-soluble coating medium. More specifically, the solid composition is preferably coated with gelatin, a yeast cell wall fraction or a cellulose derivative, for example, and then further coated with shellac, zein or the like . More preferably, the solid composition is coated with a yeast cell wall fraction and then further coated with shellac.

While the solid composition containing reduced coenzyme Q₁₀ can be coated with at least one coating medium selected from among such oil-soluble coating media and water-soluble coating media as mentioned above, it is of course possible to coat reduced coenzyme Q₁₀ alone directly or coat a solid composition prepared by any of the methods knows in the art, as mentioned above, to give a solid preparation containing reduced coenzyme Q₁₀.

On the occasion of coating the above-mentioned solid composition with such a coating medium or media as mentioned above, an auxiliary agent may be used, where necessary, for the purpose of forming suitable coatings, and the like.

The auxiliary agent is not particularly restricted provided that it is accepted for use in foods, cosmetics or drugs. One accepted for use in foods, for example a surfactant, plasticizer or coloring agent, is preferred, however.

The above-mentioned surfactants are not particularly restricted but include, for example, glycerol fatty acid esters, sucrose fatty acid esters, polysorbates and the like.

The above-mentioned plasticizers are not particularly restricted but include, for example, polyethylene glycol, glycerol, triethyl citrate, propylene glycol, vegetable oils and fats, fish oils, animal oils and fats and the like.

The above-mentioned coloring agents are not particularly restricted but include, for example, titanium oxide, food colors, bengal colors, safflower colors, caramel colors, gardenia colors, tar colors, chlorophyll and the like. In particular, titanium oxide and caramel are preferred.

The process of coating with such a coating medium as mentioned above can be carried out in a per se known manner, for example in the manner of pan coating, dry coating, oscillating coating, fluidized bed coating or the like.

The coating medium may also contain water and/or an organic solvent which can dissolve the coating medium.

The above-mentioned organic solvents are not particularly restricted in kind but include, for example, alcohols such as methanol, ethanol and 2-propanol; ketones such as acetone and methyl ethyl ketone; halogenated hydrocarbons such as chloroform and methylene chloride; hydrocarbons such as hexane, heptane and toluene; and the like. Alcohols are particularly preferred, however. Mixtures composed of two or more organic solvents, mixtures of water and an alcohol(s), mixtures of water and a ketone(s), and the like can of course be suitably used as well.

Further, the temperature at which the above coating process is carried out is not particularly restricted but, from the viewpoint of stabilization of reduced coenzyme Q₁₀ and cost, the coating can be carried out generally at a temperature not higher than about 120°C, preferably not higher than about 100°C, more preferably not higher than about 80°C, still more preferably not higher than about 60°C, most preferably not higher than about 40°C. In this case, the lower temperature limit is generally not lower than about 0°C, preferably not lower than about 10°C, more preferably not lower than about 15°C, still more preferably not lower than about 20°C, most preferably not lower than about 25°C.

The solid preparation containing reduced coenzyme Q₁₀, of the invention, as obtained in the above manner, in particular when it occurs as a powder, fine granules or granules, for example, may be suitably used without any further processing or may be modified, by compression molding, into tablets, pills or the like or, further, may be used for filling hard or soft capsules or like shells, made of gelatin or the like, therewith.

For maximizing the effects of the invention and from the viewpoint of stability of reduced coenzyme Q₁₀, the whole or a part of the production process is preferably carried out in a deoxidized atmosphere. For example, the whole production process is preferably carried out in a deoxidized atmosphere such as nitrogen gas, argon gas, helium gas or carbon dioxide.

The production method of the invention can be carried out at ordinary pressure, under increased pressure or under reduced pressure.

The weight proportion (%) of the at least one coating media selected from among oil-soluble coating media and water-soluble coating media relative to the weight (100% by weight) of the solid preparation containing reduced coenzyme Q₁₀ in the solid preparation obtained in the above manner is not particularly restricted but, from the cost viewpoint and the viewpoint of performing the function of the formed coatings, the upper limit value is generally not higher than about 99.9% by weight, preferably not higher than about 90% by weight, still more preferably not higher than about 80% by weight, still further preferably not higher than about 70% by weight. The lower limit value is generally not lower than about 5% by weight, preferably not lower than about 10% by weight, more preferably not lower than about 15% by weight, still more preferably not lower than about 20% by weight, most preferably not lower than about 25% by weight. The above-mentioned weight of the coating media is that determined after formulation.

The content of reduced coenzyme Q₁₀ in the solid preparation is not particularly restricted but, from the effectiveness, and the like, of reduced coenzyme Q₁₀ and easiness of formulation viewpoint, it is preferably 0.1 to 95% by weight, more preferably 1 to 90% by weight.

The solid preparation of the invention, when preserved in the air at 40°C in a condition shielded from light for 30 days, shows a reduced coenzyme Q₁₀ retention percentage (percentage of the weight of reduced coenzyme Q₁₀ after preservation to the initial weight of reduced coenzyme Q₁₀) of not lower than about 50% by weight, preferably not lower than about 60% by weight, still more preferably not lower than about 70% by weight, still further preferably not lower than about 80% by weight, most preferably not lower than about 90% by weight.

From the viewpoint of stabilization of reduced coenzyme Q₁₀, the upper limit to the temperature at which the solid preparation of the invention is to be preserved is generally not higher than about 100°C, preferably not higher than about 80°C, still more preferably not higher than about 60°C, still further preferably not higher than about 40°C, most preferably not higher than about 20C. The lower limit to that temperature is generally not lower than about -100°C, preferably not lower than about -80°C, still more preferably not lower than about -60°C, still further preferably not lower than about -40°C, most preferably not lower than about -20°C.

The humidity of the atmosphere in which the preparation is to be preserved is not particularly restricted but, from the viewpoint of stabilization of reduced coenzyme Q₁₀, it is generally not higher than about 90%, preferably not higher than about 80%, more preferably not higher than about 75%, still more preferably not higher than about 60%, still further preferably not higher than about 40%, and the lower limit thereof is not lower than 0%, as expressed in terms of relative humidity.

Now, the method for stabilizing and the method for handling a solid preparation containing reduced coenzyme Q₁₀ according to the invention are described.

The method for stabilizing a solid preparation containing reduced coenzyme Q₁₀ according to the disclosure is characterized in that a solid composition containing reduced coenzyme Q₁₀ is coated with at least one coating medium selected from among oil-soluble coating media and water-soluble coating media to thereby stabilize the resulting solid preparation containing reduced coenzyme Q₁₀.

The method for handling a solid preparation containing reduced coenzyme Q₁₀ according to the invention is characterized in that the solid preparation containing reduced coenzyme Q₁₀ is placed in an environment adjusted to a relative humidity of not higher than 75%.

The stabilization, so referred to herein, indicates the inhibition of the oxidation of reduced coenzyme Q₁₀ to oxidized coenzyme Q₁₀.

The handling, so referred to herein, is the exertion of an external action on something to cause the same to maintain or perform some or other function thereof. Examples of the handling are not particularly restricted but may include discharging from the coating machine, packaging, packing, preservation, storage, transfer and the like. A preferred handling consists in preservation.

From the viewpoint of stability of reduced coenzyme Q₁₀, the upper limit to the temperature at which the solid preparation containing reduced coenzyme Q₁₀, of the invention, is to be preserved or handled in according to the method for stabilizing and the method for handling that preparation is generally not higher than about 100ºC, preferably not higher than about 80ºC, still more preferably not higher than about 60ºC, still further preferably not higher than about 40ºC, most preferably not higher than about 20ºC. In this case, the lower limit to that temperature is generally not lower than about -100ºC, preferably not lower than about -80ºC, still more preferably not lower than about -60°C, still further preferably not lower than about -40°C, most preferably not lower than about -20°C.

The solid preparation of the invention, when preserved in the air at 40°C in a condition shielded from light for 30 days, shows a reduced coenzyme Q₁₀ retention percentage of not lower than about 50% by weight, preferably not lower than about 60% by weight, still more preferably not lower than about 70% by weight, still further preferably not lower than about 80% by weight, most preferably not lower than about 90% by weight.

In practicing the method for stabilizing and the method for handling the solid preparation containing reduced coenzyme Q₁₀ according to the invention, the humidity in the preserving atmosphere is important and, by controlling that humidity, it becomes possible to markedly improve the stability of the solid preparation containing reduced coenzyme Q₁₀ The upper limit to the relative humidity is generally not higher than about 90%, preferably not higher than about 80%, more preferably not higher than about 75%, still more preferably not higher than about 60%, still further preferably not higher than about 40%, and, in such a controlled environment, the solid preparation containing reduced coenzyme Q₁₀ can be handled in a more stable condition. The lower limit to the relative humidity is not lower than 0%.

In a preferred mode of embodiment in which the humidity in the preserving atmosphere is controlled in the above manner, the reduced coenzyme Q₁₀ retention percentage after 30 days of preservation in the air at 40°C in a condition shielded from light is not lower than about 80% by weight, preferably not lower than about 85% by weight, more preferably not lower than about 90% by weight, still more preferably not lower than about 95% by weight, most preferably not lower than about 97% by weight.

Such an environment with a controlled relative humidity can be given, for example, by dehumidification from the environment; introduction of a dehumidified gas (preferably a dry inert gas, though air may also be employed) into the environment; and the like. The method of dehumidification is not particularly restricted but the dehumidification may be accomplished by the freezing of moisture or the use of a dehumidifier or a desiccant (e. g. silica gel, calcium chloride, synthetic zeolite), and the like. It goes without saying that if an environment having a controlled relative humidity is given, the method for creating the same does not matter in any way.

For maximizing the effects of the invention and from the viewpoint of the stability of reduced enzyme Q₁₀, the preservation and/or handling of the solid preparation is preferably carried out in a deoxidized atmosphere, as a matter of course. Thus, for example, the present invention is preferably practiced in a deoxidized atmosphere such as an inert gas, for example nitrogen gas, argon gas, helium gas, carbon dioxide or the like.

The solid preparation containing reduced coenzyme Q₁₀ as obtained according to the invention can be handled, for example packaged and packed, using a glass bottle, a plastic bottle, a plastic bag, an aluminum-laminated bag or the like. As the materials to be used in the above-mentioned handling, for example packaging or packing, there may be mentioned glass, high-density polyethylene, medium-density polyethylene, low-density polyethylene, polyethylene terephthalate, polyvinyl alcohol, polypropylene, polyvinyl chloride, polyvinylidene chloride and like materials. Metal (e.g. aluminum) film-based materials manufactured by lamination with any of the above-mentioned grades of polyethylene or polyethylene terephthalate, for example, can also be suitably used. When a material relatively inferior in gas barrier performance and moistureproofing performance, for example polyethylene, is used, it is desirable that at least double packaging or packing be done using an outer bag made of a material excellent in gas barrier performance and moistureproofing performance, for example an aluminum-laminate film.

It is also possible to carry out PTP packaging, three side-sealed packaging, four side-sealed packaging, pillow packaging, strip packaging, aluminum shaped packaging, stick packaging or the like using the materials mentioned above. After packaging/packing, the packages/packs obtained can be enclosed, if necessary, in a steel drum, resin drum, fiber drum, corrugated fiberboard box or like container for transportation and/or storage. It is of course possible to enclose a desiccant such as silica gel, calcium chloride or a synthetic zeolite in such a container.

### (EFFECT OF THE INVENTION)

In accordance with the present invention, reduced coenzyme Q₁₀ which is unstable in the air can be maintained very stably.

### BRIEF DESCRIPTION OF THE DRAWINGS

Fig. 1 shows the weight proportions (%) of reduced coenzyme Q₁₀ after 15 days and 30 days of preservation of the coated tablets produced in Example 1 in the following adjusted environments: shielded from light, in the air, 40ºC, relative humidity 10%, 40%, 60% and 75%.
Fig. 2 shows the weight proportions (%) of reduced coenzyme Q₁₀ after 15 days and 30 days of preservation of the coated tablets produced in Example 2 in the following adjusted environments: shielded from light, in the air, 40ºC, relative humidity 10%, 40%, 60% and 75%.

### BEST MODE FOR CARRYING OUT THE INVENTION

The following examples 1, 2 and 6 to 8 illustrate the invention in further detail. They are, however, by no means limitative of the scope of the invention.

The purity of reduced coenzyme Q₁₀ and the weight proportion (%)of reduced coenzyme Q₁₀ were determined by the following HPLC analysis (weight proportion (%) = reduced coenzyme Q₁₀/(oxidized coenzyme Q₁₀ + reduced coenzyme Q₁₀) x 100).

### (HPLC analysis conditions)

Column: SYMMETRY C18 (product of Waters Corporation), 250 mm (length), 4.6 mm (inside diameter) ; mobile phase: C₂H₅OH/CH₃OH = 4/3 (v/v); detection wavelength: 210 nm; flow rate: 1.0 ml/min; retention time of reduced coenzyme Q₁₀: 9.1 min; retention time of oxidized coenzyme Q₁₀: 13.3 min.

### (Synthesis Example 1)

Crystals of oxidized coenzyme Q₁₀ (100 g) and 60 g of L-ascorbic acid were added to 1000 g of ethanol, and the reduction reaction was carried out with stirring at 78°C. After the lapse of 30 hours, the mixture was cooled to 50°C and, while maintaining that temperature, 400 g of ethanol and 100 g of water were added. The resulting ethanol solution was cooled to 2°C at a rate of 10°C/hour with stirring. The precipitate was washed in sequence with cold ethanol and cold water, and the wet crystals obtained were dried under reduced pressure to give 95 g of dry white crystals (yield after isolation: 95 mole %). All the procedural steps except for vacuum drying were carried out in a nitrogen atmosphere. The purity of the crystals obtained was 99.1%, and the weight proportion (%) of reduced coenzyme Q₁₀ was 99.0%.

### (Synthesis Example 2)

The reduced coenzyme Q₁₀ crystals obtained in Synthesis Example 1, crystalline cellulose (AVICEL(R)), cornstarch and lactose were mixed up according to the formulation given below and, further, magnesium stearate (1 part by weight) was admixed with the mixture obtained, to give a mixed powder. The particle size of the mixed powder obtained was adjusted by sifting, and the sieved powder was tableted on a rotary tablet machine to give uncoated tablets each having a total weight of 150 mg and containing 30 mg of reduced coenzyme Q₁₀.

| | |
|---|---|
| Reduced coenzyme Q₁₀ crystal | 20 weight parts |
| Crystalline cellulose (AVICEL(R)) | 20 weight parts |
| Cornstarch | 20 weight parts |
| Lactose | 39 weight parts |

### (Example 1)

The uncoated tablets containing reduced coenzyme Q₁₀ as obtained in Synthesis Example 2 were sprayed with a solution composed of 500 g of an aqueous solution of a yeast cell wall fraction (product of KIRIN BREWERY CO., LTD., YeastWrap(R)) and 4 g of glycerol, followed by drying. Thus was produced a reduced coenzyme Q₁₀-containing solid preparation (coated tablets) coated with about 50 mg, per tablet, of the yeast cell wall fraction.

### (Example 2)

The uncoated tablets containing reduced coenzyme Q₁₀ as obtained in Synthesis Example 2 were sprayed with a solution composed of 450 g of purified water and 50 g of gelatin (product of Nitta Gelatin Inc., APH-100), followed by drying. Thus was produced a reduced coenzyme Q₁₀-containing solid preparation (coated tablets) coated with about 50 mg, per tablet, of gelatin.

### (Example 3)

The uncoated tablets containing reduced coenzyme Q₁₀ as obtained in Synthesis Example 2 were sprayed with a solution composed of 450 g of purified water and 50 g of hydroxypropylmethylcellulose (product of Shin-Etsu Chemical Co., Ltd., Metolose 90SH-04)), followed by drying. Thus was produced a reduced coenzyme Q₁₀-containing solid preparation (coated tablets) coated with about 50 mg, per tablet, of hydroxypropylmethylcellulose.

### (Example 4)

The uncoated tablets containing reduced coenzyme Q₁₀ as obtained in Synthesis Example 2 were sprayed with an ethanol solution of shellac (product of GIFU SHELLAC), followed by drying. Thus was produced a reduced coenzyme Q₁₀-containing solid preparation (coated tablets) coated with about 30 mg, per tablet, of shellac.

### (Example 5)

The uncoated tablets containing reduced coenzyme Q₁₀ as obtained in Synthesis Example 2 were sprayed with an ethanol solution of shellac (product of GIFU SHELLAC), followed by drying. The tablets thus provided with a moistureproofing coating were further sprayed with an aqueous solution composed of 44 g of purified water, 44 g of purified sucrose and 12 g of gum arabic (product of Ina Food Industry Co., Ltd., Gum Arabic A), followed by drying. Thus was produced a reduced coenzyme Q₁₀-containing solid preparation (sugar-coated tablets) coated with about 80 mg, per tablet, of purified sucrose and gum arabic.

### (Example 6)

The uncoated tablets containing reduced coenzyme Q₁₀ as obtained in Synthesis Example 2 were sprayed with a solution composed of 500 g of an aqueous solution of a yeast cell wall fraction (product of KIRIN BREWERY CO., LTD., YeastWrap (R)) and 4 g of glycerol, followed by drying. Thus was produced a reduced coenzyme Q₁₀-containing solid preparation (coated tablets) coated with about 50 mg, per tablet, of the yeast cell wall fraction. Further, this solid preparation was sprayed with an ethanol solution of shellac (product of GIFU SHELLAC), followed by drying. Thus was produced a reduced coenzyme Q₁₀-containing solid preparation (coated tablets) coated with about 2 mg, per tablet, of shellac.

### (Example 7)

Reduced coenzyme Q₁₀-containing granules were prepared by mixing the reduced coenzyme Q₁₀ crystals obtained in Synthesis Example 1, hydroxypropylcellulose, lactose and ethanol together according to the formulation shown below, stirring and drying the mixture. Those granules were sprayed with a solution composed of 500 g of an aqueous solution of a yeast cell wall fraction (product of KIRIN BREWERY CO., LTD., YeastWrap(R)) and 4 g of glycerol, followed by drying. Thus was produced a reduced coenzyme Q₁₀-containing solid preparation (granules) coated with about 30 g of the yeast cell wall fraction per 70 g of the granules. Magnesium stearate (1 part by weight per 100 parts by weight of the granules) was admixed with this solid preparation (granules) and the resulting mixed powder was tableted on a rotary tablet machine to give reduced coenzyme Q₁₀-containing tablets each having a total weight of 200 mg and containing 30 mg of reduced coenzyme Q₁₀.

| | |
|---|---|
| Reduced coenzyme Q₁₀ crystal | 20 weight parts |
| Hydroxypropylcellulose | 6 weight parts |
| Lactose | 73 weight parts |
| Ethanol | 50 weight parts |

### (Example 8)

The reduced coenzyme Q₁₀ crystals obtained in Synthesis Example 1, crystalline cellulose (AVICEL(R)), cornstarch and purified sucrose were mixed up according to the formulation given below and, further, magnesium stearate (1 part by weight) was admixed with the mixture obtained, to give a mixed powder. The particle size of the mixed powder obtained was adjusted by sifting, and the sieved powder was tableted on a rotary tablet machine to give chewable tablets each having a total weight of 500 mg and containing 100 mg of reduced coenzyme Q₁₀. Those chewable tablets were sprayed with a solution composed of 500 g of an aqueous solution of a yeast cell wall fraction (product of KIRIN BREWERY CO., LTD., YeastWrap(R)) and 4 g of glycerol, followed by drying. Thus was produced a reduced coenzyme Q₁₀-containing solid preparation (chewable tablets) coated with about 170 mg, per tablet, of the yeast cell wall fraction. The solid preparation was further sprayed with an ethanol solution of shellac (product of GIFU SHELLAC), followed by drying. Thus was produced a reduced coenzyme Q₁₀-containing solid preparation (coated tablets) coated with about 40 mg, per tablet, of shellac.

| | |
|---|---|
| Reduced coenzyme Q₁₀ crystal | 20 weight parts |
| Crystalline cellulose (AVICEL(R)) | 10 weight parts |
| Cornstarch | 5 weight parts |
| Purified sucrose | 64 weight parts |

### (Example 9)

The coated tablets produced in Examples 1 to 4 and 6 to 8 were preserved in the following adjusted environment: shielded from light, in the air, 40°C and relative humidity 60%. After 15 days and 30 days, the weight proportions (%) of reduced coenzyme Q₁₀ were determined by the above-mentioned HPLC analysis. The results are shown in Table 1. For comparison, the results obtained with the uncoated tables produced in Synthesis Example 2 as a control are also shown. In evaluating the results, reduced coenzyme Q₁₀ retention percentages of not lower than about 80% by weight after 30 days of preservation in the adjusted environment (shielded from light, in the air, 40°C, relative humidity 60%) were regarded as indicative of successful stabilization.

**Table 1**

| | Coating medium | Weight proportion (%) of reduced coenzyme Q10 | |
|---|---|---|---|
| | | After 15 days | After 30 days |
| Example 1 | Yeast cell wall | 100% | 100% |
| Example 2 | Gelatin | 100% | 100% |
| Example 3 | Hydroxypropylmethylcellulose | 94% | 90% |
| Example 4 | Shellac | 97% | 94% |
| Example 6 | Yeast cell wall+Shellac | 100% | 100% |
| Example 7 | Yeast cell wall | 100% | 100% |
| Example 8 | Yeast cell wally+Shellac | 100% | 100% |
| Control | Uncoated tablets | 67% | 45% |

### (Example 10)

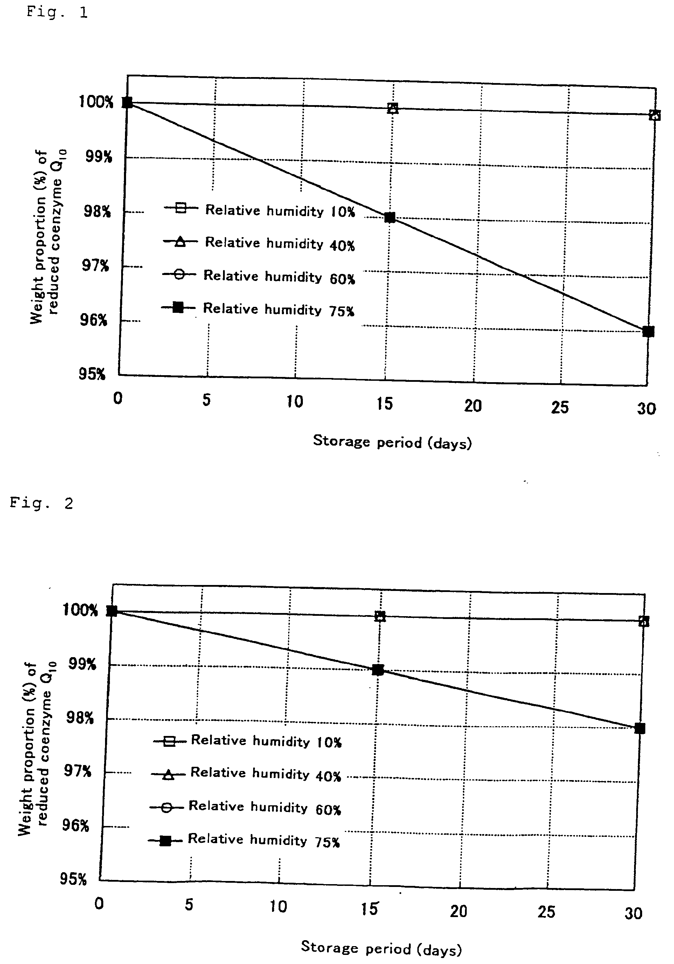

The coated tablets produced in Example 1 were preserved in the following adjusted environments: shielded from light, in the air, 40°C, relative humidity 10%, 40%, 60% and 75%. After 15 days and 30 days, the weight proportions (%) of reduced coenzyme Q₁₀ were determined by the above-mentioned HPLC analysis. The results are shown in Fig. 1. In evaluating the results, reduced coenzyme Q₁₀ retention percentages of not lower than about 80% by weight after 30 days of preservation in the adjusted environment (shielded from light, in the air, 40°C, each of relative humidity) were regarded as indicative of successful stabilization.

### (Example 11)

The coated tablets produced in Example 2 were preserved in the following adjusted environments: shielded from light, in the air, 40°C, relative humidity 10%, 40%, 60% and 75%. After 15 days and 30 days, the weight proportions (%) of reduced coenzyme Q₁₀ were determined by the above-mentioned HPLC analysis. The results are shown in Fig. 2. In evaluating the results, reduced coenzyme Q₁₀ retention percentages of not lower than about 80% by weight after 30 days of preservation in the adjusted environment (shielded from light, in the air, 40°C, each of relative humidity) were regarded as indicative of successful stabilization.

### INDUSTRIAL APPLICABILITY

In accordance with the present invention, reduced coenzyme Q₁₀ which is unstable in the air can be maintained very stably.

## Claims

1. A solid preparation containing reduced coenzyme Q₁₀
which comprises a solid composition containing reduced coenzyme Q₁₀ coated with at least one water-soluble coating medium, which is at least one species selected from the group consisting of gelatin and yeast cell wall fractions.

2. The solid preparation according to Claim 1,
wherein the solid composition containing reduced coenzyme Q₁₀ is coated with a water-soluble coating medium of gelatin and/or yeast cell wall fractions and then further with an oil-soluble coating medium.

3. The solid preparation according to Claim 1,
wherein the coating medium is that accepted for use in foods.

4. The solid preparation according to Claim 2,
wherein the oil-soluble coating medium used is at least one species selected from among shellac and zein.

5. The solid preparation according to Claim 1,
wherein the total coating weight of the coating medium or media is not less than 5% by weight but not more than 99.9% by weight relative to the weight of the solid preparation.

6. The solid preparation according to Claim 1,
which shows a percent retention of reduced coenzyme Q₁₀ of not lower than 50% by weight after 30 days of preservation in the air at 40°C in a condition protected from light.

7. A method for producing a solid preparation containing reduced coenzyme Q₁₀,
wherein a solid composition containing reduced coenzyme Q₁₀ is coated with at least one water-soluble coating medium, which is one species selected from the group consisting of gelatin and yeast cell wall fractions.

8. The production method according to Claim 7,
wherein the solid composition containing reduced coenzyme Q₁₀ is coated with a water-soluble coating medium of gelatin and/or yeast cell wall fractions and then further with an oil-soluble coating medium.

9. The production method according to Claim 7,
wherein the coating medium is that accepted for use in foods.

10. The production method according to Claim 8,
wherein the oil-soluble coating medium used is at least one species selected from among shellac and zein.

11. The production method according to Claim 7,
wherein the coating is carried out at a temperature of not lower than 0°C but not higher than 120°C.

12. A method for stabilizing a solid preparation containing reduced coenzyme Q₁₀,
wherein a solid composition containing reduced coenzyme Q₁₀ is coated with at least one water-soluble coating medium, which is one species selected from the group consisting of gelatin and yeast cell wall fractions, to thereby stabilize the resulting solid preparation containing reduced coenzyme Q₁₀.

13. The stabilization method according to Claim 12,
wherein the solid composition containing reduced coenzyme Q₁₀ is coated with a water-soluble coating medium of gelatin and/or yeast cell wall fractions and then further with an oil-soluble coating medium.

14. The stabilization method according to Claim 12,
wherein the coating medium is that accepted for use in foods.

15. The stabilization method according to Claim 13,
wherein the oil-soluble coating medium used is at least one species selected from among shellac and zein.

16. The stabilization method according to Claim 12,
wherein the percent retention of reduced coenzyme Q₁₀ is not lower than 50% by weight after 30 days of preservation in the air at 40°C in a condition protected from light.

17. A method for handling a solid preparation containing reduced coenzyme Q₁₀,
wherein the solid preparation containing reduced coenzyme Q₁₀ according to Claim 1 is placed in an environment adjusted to a relative humidity of not higher than 75%.

18. The handling method according to Claim 17,
wherein the percent retention of reduced coenzyme Q₁₀ is not lower than 80% by weight after 30 days of preservation in the air at 40°C in a condition protected from light.

## Patentansprüche

1. Reduziertes Coenzym Q₁₀ enthaltende Feststoffzubereitung,
die eine reduziertes Coenzym Q₁₀ enthaltende Feststoffzusammensetzung umfasst, die mit wenigstens einem wasserlöslichen Beschichtungsmedium beschichtet ist, das wenigstens eine Spezies ist, die aus der aus Gelatine und Hefezellwandfraktionen bestehenden Gruppe ausgewählt ist.

2. Feststoffzubereitung nach Anspruch 1,
wobei die reduziertes Coenzym Q₁₀ enthaltende Feststoffzusammensetzung mit einem wasserlöslichen Beschichtungsmedium aus Gelatine und/oder Hefezellwandfraktionen und dann weiter mit einem öllöslichen Beschichtungsmedium beschichtet ist.

3. Feststoffzubereitung nach Anspruch 1,
wobei das Beschichtungsmedium das ist, das zur Verwendung in Lebensmitteln zugelassen ist.

4. Feststoffzubereitung nach Anspruch 2,
wobei das verwendete öllösliche Beschichtungsmedium wenigstens eine Spezies ist, die aus Schellack und Zein ausgewählt ist.

5. Feststoffzubereitung nach Anspruch 1,
wobei das Gesamtbeschichtungsgewicht des/r Beschichtungsmediums oder -medien nicht weniger als 5 Gewichts-% aber nicht mehr als 99,9 Gewichts-% in Bezug auf das Gewicht der Feststoffzubereitung beträgt.

6. Feststofifzubereitung nach Anspruch 1,
die eine prozentuale Aufrechterhaltung des reduzierten Coenzyms Q₁₀ von nicht weniger als 50 Gewichts-% nach 30 Tagen Aufbewahrung unter lichtgeschützten Bedingungen an der Luft bei 40 °C aufweist.

7. Verfahren zum Herstellen einer reduziertes Coenzym Q₁₀ enthaltenden Feststoffzubereitung,
wobei eine reduziertes Coenzym Q₁₀ enthaltende Feststofifzusammensetzung mit wenigstens einem wasserlöslichen Beschichtungsmedium beschichtet ist, das eines ist, das aus der aus Gelatine und Hefezellwandfraktionen bestehenden Gruppe ausgewählt ist.

8. Herstellungsverfahren nach Anspruch 7,
wobei die reduziertes Coenzym Q₁₀ enthaltende Feststoffzusammensetzung mit einem wasserlöslichen Beschichtungsmedium aus Gelatine und/oder Hefezellwandfraktionen und dann weiter mit einem öllöslichen Beschichtungsmedium beschichtet ist.

9. Herstellungsverfahren nach Anspruch 7,
wobei das Beschichtungsmedium das ist, das zur Verwendung in Lebensmitteln zugelassen ist.

10. Herstellungsverfahren nach Anspruch 8,
wobei das verwendete öllösliche Beschichtungsmedium wenigstens eines ist, das aus Schellack und Zein ausgewählt ist.

11. Herstellungsverfahren nach Anspruch 7,
wobei die Beschichtung bei einer Temperatur von nicht weniger als 0 °C aber nicht mehr als 120 °C durchgeführt wird.

12. Verfahren zum Stabilisieren einer reduziertes Coenzym Q₁₀ enthaltenden Feststoffzubereitung,
wobei eine reduziertes Coenzym Q₁₀ enthaltende Feststoffzusammensetzung mit wenigstens einem wasserlöslichen Beschichtungsmedium beschichtet ist, das eines ist, das aus der aus Gelatine und Hefezellwandfraktionen bestehenden Gruppe ausgewählt ist, um dadurch die resultierende reduziertes Coenyzm Q₁₀ enthaltende Feststoffzubereitung zu stabilisieren.

13. Stabilisierungsverfahren nach Anspruch 12,
wobei die reduziertes Coenzym Q₁₀ enthaltende Feststoffzusammensetzung mit einem wasserlöslichen Beschichtungsmedium aus Gelatine und/oder Hefezellwandfraktionen und dann weiter mit einem öllöslichen Beschichtungsmedium beschichtet ist.

14. Stabilisierungsverfahren nach Anspruch 12,
wobei das Beschichtungsmedium das ist, das zur Verwendung in Lebensmitteln zugelassenist.

15. Stabilisierungsverfahren nach Anspruch 13,
wobei das verwendete öllösliche Beschichtungsmedium wenigstens eines ist, das aus Schellack und Zein ausgewählt ist.

16. Stabilisierungsverfahren nach Anspruch 12,
wobei die prozentuale Aufrechterhaltung des reduzierten Coenzyms Q₁₀ nicht weniger als 50 Gewichts-% nach 30 Tagen Aufbewahrung unter lichtgeschützten Bedingungen an der Luft bei 40 °C beträgt.

17. Verfahren zur Handhabung einer reduziertes Coenzym Q₁₀ enthaltenden Feststoffzubereitung,
wobei die reduziertes Coenzym Q₁₀ enthaltende Feststoffzubereitung nach Anspruch 1 in einer Umgebung eingesetzt wird, die auf eine relative Feuchtigkeit von nicht mehr als 75 % eingestellt ist.

18. Handhabungsverfahren nach Anspruch 17,
wobei die prozentuale Aufrechterhaltung des reduzierten Coenzyms Q₁₀ nicht weniger als 80 Gewichts-% nach 30 Tagen Aufbewahrung unter lichtgeschützten Bedingungen an der Luft bei 40 °C beträgt.

## Revendications

1. Préparation solide contenant une co-enzyme Q₁₀ réduite
laquelle comprend une composition solide contenant une co-enzyme Q₁₀ réduite revêtue avec au moins un milieu de revêtement soluble dans l'eau, lequel est au moins une espèce choisie dans le groupe constitué de fractions de parois cellulaires de gélatine et de levure.

2. Préparation solide selon la revendication 1,
dans laquelle la composition solide comprenant une co-enzyme Q₁₀ réduite est revêtue avec un milieu de revêtement soluble dans l'eau de fractions de parois cellulaires de gélatine et/ou de levure et ensuite de plus avec un milieu de revêtement soluble dans l'huile.

3. Préparation solide selon la revendication 1,
dans laquelle le milieu de revêtement est celui accepté pour une utilisation dans des aliments.

4. Préparation solide selon la revendication 2,
dans laquelle le milieu de revêtement soluble dans l'huile utilisé est au moins une espèce choisie parmi le shellac et la zéine.

5. Préparation solide selon la revendication 1,
dans laquelle la masse de revêtement totale du milieu ou des milieux de revêtement n'est pas inférieure à 5 % en masse mais n'est pas supérieure à 99,9 % en masse par rapport à la masse de la préparation solide.

6. Préparation solide selon la revendication 1,
laquelle présente un pourcentage de rétention de co-enzyme Q₁₀ réduite qui n'est pas inférieur à 50 % en masse après 30 jours de conservation dans l'air à 40°C dans un état protégé de la lumière.

7. Procédé de production d'une préparation solide contenant une co-enzyme Q₁₀ réduite,
dans lequel une composition solide contenant une co-enzyme Q₁₀ réduite est revêtue avec au moins un milieu de revêtement soluble dans l'eau qui est une espèce choisie dans le groupe constitué de fractions de parois cellulaires de gélatine et de levure.

8. Procédé de production selon la revendication 7,
dans lequel la composition solide contenant une co-enzyme Q₁₀ réduite est revêtue avec un milieu de revêtement soluble dans l'eau de fractions de parois cellulaires de gélatine et/ou de levure et ensuite de plus avec un milieu de revêtement soluble dans l'eau.

9. Procédé de production selon la revendication 7,
dans lequel le milieu de revêtement est celui accepté pour une utilisation dans des aliments.

10. Procédé de production selon la revendication 8,
dans lequel le milieu de revêtement soluble dans l'huile utilisé est au moins une espèce choisie parmi le shellac et la zéine.

11. Procédé de production selon la revendication 7,
dans lequel le revêtement est réalisé à une température qui n'est pas inférieure à 0°C mais pas supérieure à 120°C.

12. Procédé de stabilisation d'une préparation solide contenant une co-enzyme Q₁₀ réduite,
dans lequel une composition solide contenant une co-enzyme réduite Q₁₀ est revêtue avec au moins un milieu de revêtement soluble dans l'eau, qui est une espèce choisie dans le groupe constitué de fractions de parois cellulaires de gélatine et de levure, pour stabiliser par-là la préparation solide résultante contenant une co-enzyme Q₁₀ réduite.

13. Procédé de stabilisation selon la revendication 12,
dans lequel la composition solide contenant une co-enzyme Q₁₀ réduite est revêtue avec un milieu de revêtement soluble dans l'eau de fractions de parois cellulaires de gélatine et/ou de levure et ensuite de plus avec un milieu de revêtement soluble dans l'eau.

14. Procédé de stabilisation selon la revendication 12,
dans lequel le milieu de revêtement est celui accepté pour une utilisation dans des aliments.

15. Procédé de stabilisation selon la revendication 13,
dans lequel le milieu de revêtement soluble dans l'huile utilisé est au moins une espèce choisie parmi le shellac et la zéine.

16. Procédé de stabilisation selon la revendication 12,
dans lequel le pourcentage de rétention de co-enzyme Q₁₀ réduite n'est pas inférieur à 50 % en masse après 30 jours de conservation à l'air à 40°C dans un état protégé de la lumière.

17. Procédé de manipulation d'une préparation solide contenant une co-enzyme Q₁₀ réduite,
dans lequel la préparation solide contenant une co-enzyme Q₁₀ réduite selon la revendication 1 est placée dans un environnement ajusté à une humidité relative qui n'est pas supérieure à 75 %.

18. Procédé de manipulation selon la revendication 17,
dans lequel le pourcentage de rétention de co-enzyme Q₁₀ réduite n'est pas inférieur à 80 % en masse après 30 jours de conservation dans l'air à 40°C dans un état protégé de la lumière.
